(19) Europäisches Patentamt
European Patent Office
Office européen des brevets

(11) **EP 0 805 794 B1**

(12) **EUROPÄISCHE PATENTSCHRIFT**

(45) Veröffentlichungstag und Bekanntmachung des Hinweises auf die Patenterteilung:
**05.09.2001 Patentblatt 2001/36**

(21) Anmeldenummer: **96901284.8**

(22) Anmeldetag: **17.01.1996**

(51) Int Cl.[7]: **C07C 229/16**, C07C 217/50, C07C 227/02

(86) Internationale Anmeldenummer:
**PCT/EP96/00169**

(87) Internationale Veröffentlichungsnummer:
**WO 96/22964 (01.08.1996 Gazette 1996/35)**

(54) **VERWENDUNG VON TRISÄUREN AUF BASIS ALKOXYLIERTER TERTIÄRER AMINE ALS KOMPLEXBILDNER**

USE OF TRI-ACIDS BASED ON ALKOXYLATED TERTIARY AMINES AS COMPLEX FORMERS

UTILISATION DE TRIACIDES A BASE D'AMINES TERTIAIRES ALCOXYLEES COMME COMPLEXANTS

(84) Benannte Vertragsstaaten:
**AT BE CH DE DK ES FR GB IT LI NL SE**

(30) Priorität: **26.01.1995 DE 19502294**

(43) Veröffentlichungstag der Anmeldung:
**12.11.1997 Patentblatt 1997/46**

(73) Patentinhaber: **BASF AKTIENGESELLSCHAFT
67056 Ludwigshafen (DE)**

(72) Erfinder:
- **GREINDL, Thomas
D-94127 Neuburg (DE)**
- **POTTHOFF-KARL, Birgit
D-67061 Ludwigshafen (DE)**
- **OFTRING, Alfred
D-67098 Bad Dürkheim (DE)**
- **FETZER, Thomas
D-67346 Speyer (DE)**
- **BAUR, Richard
D-67112 Mutterstadt (DE)**
- **TRIESELT, Wolfgang
D-76135 Karlsruhe (DE)**

(56) Entgegenhaltungen:
**GB-A- 1 122 390** **US-A- 2 316 636**

- **CHEMICAL ABSTRACTS, vol. 121, no. 22, 28.November 1994 Columbus, Ohio, US; abstract no. 267696, OKADA, HISASHI: "Photographic processing compositions containing nitrogen compound metal chelates" XP002003314 & JP,A,06 180 483 (FUJI PHOTO FILM CO LTD, JAPAN) & STN Registry File RN 158877-07-7**
- **SUPRAMOLECULAR CHEMISTRY , Bd. 2, 1993, Seiten 247-250, XP000571128 K. RISSANEN ET AL.: "Self-assembly by coordination and strong hydrogen bonding. X-ray crystal structures of a dimeric trisodium complex of a new acidic complexing ligand and its dihydrate"**
- **CHEMICAL ABSTRACTS, vol. 123, no. 16, 16.Oktober 1995 Columbus, Ohio, US; abstract no. 212991, OKADA, HISASHI ET AL: "Photographic processing composition and processing method" XP002003313 & JP,A,07 120 894 (FUJI PHOTO FILM CO LTD, JAPAN) & STN Registry File RN 167769-55-3 RN 167769-49-5**
- **CHEMICAL ABSTRACTS, vol. 123, no. 18, 30.Oktober 1995 Columbus, Ohio, US; abstract no. 241860, OKADA, HISASHI ET AL: "Photographic processing composition containing aminepolycarboxylic acid compound and processing method" XP002003315 & JP,A,07 120 899 (FUJI PHOTO FILM CO LTD, JAPAN) & STN Registry File RN 167769-49-5**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 805 794 B1

Printed by Jouve, 75001 PARIS (FR)

## Beschreibung

Die vorliegende Erfindung betrifft die Verwendung von Trisäuren der allgemeinen Formel I

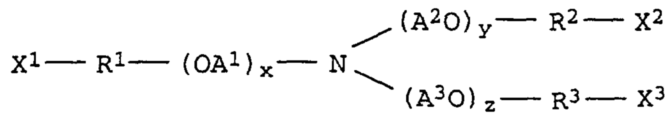

(I)

in der

| | |
|---|---|
| $X^1$ bis $X^3$ | Carbonsäuregruppen der Formel COOM bedeuten, wobei |
| M | für Wasserstoff, Alkalimetall, Ammonium oder substituiertes Ammonium steht, |
| $A^1$ bis $A^3$ | unabhängig voneinander 1,2-Alkylen mit 2 bis 18 C-Atomen bezeichnen, |
| $R^1$ bis $R^3$ | unabhängig voneinander Methylen, 1,1-Ethylen oder 1,1-Propylen bedeuten und |
| x, y und z | unabhängig voneinander für eine Zahl von 1 bis 10 stehen, |

als Schwermetall-Komplexbildner in Bleichbädern in der Papierindustrie, in Wasch- und Reinigungsmitteln, in kosmetischen oder pharmazeutischen Formulierungen, bei der Seifenherstellung, in der Pflanzenernährung sowie zur Herstellung von Schwermetallkomplexen zum Einsatz in galvanischen Bädern oder bei der Rauchgasentschwefelung.

Als Komplexbildner für Schwermetallionen auf den verschiedensten technischen Gebieten mit ihren teilweise stark voneinander abweichenden Anforderungs- und Problemfeldern werden üblicherweise immer noch altbekannte und bewährte Systeme wie Polyphosphate, Nitrilotriessigsäure oder Ethylendiamintetraessigsäure eingesetzt. Diese Mittel zeigen allerdings gewisse Nachteile, prinzipielle Schwachpunkte sind insbesondere ihr noch verbesserungsbedürftiges Schwermetall-Bindevermögen, ihre noch nicht optimale stabilisierende Wirkung in Bleichbädern und Bleichsystemen sowie ihre meist unzureichende biologische Abbaubarkeit bzw. Eliminierbarkeit.

In der US-A 2 316 636 wird ein Verfahren zur Herstellung von Aminopolycarbonsäuren und deren Salzen aus den zugrunde liegenden Alkoholaminen beschrieben. Als einzelne Aminopolycarbonsäuren werden u.a. die Verbindungen

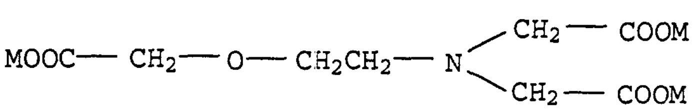

,

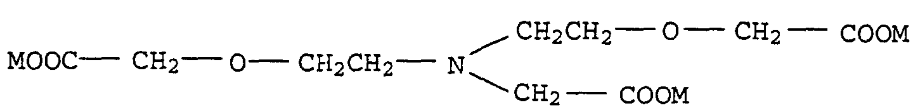

und

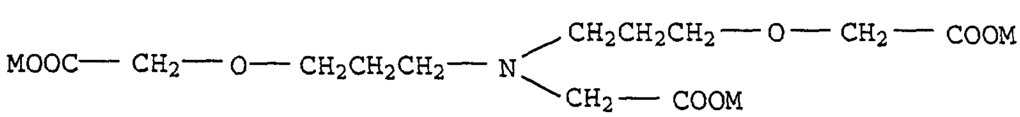

genannt. Derartige Aminopolycarbonsäuren werden für die technischen Gebiete der Waschmittel und Waschhilfsmittel, der Wasserenthärtung, der Wasserbehandlung und der Textilbleiche empfohlen.

Aus Chem. Abs., 121: Abs. 267696d (RN 158877-07-7) ist eine zu den vorliegenden Verbindungen I analoge Verbindung bekannt, welche allerdings nur in einer Kette am N-Atom Alkylenoxid-Einheiten aufweist ($X^1$, $X^2$, $X^3$ = COOH, $R^1$ bis $R^3$ = $CH_2$, x=y=0, z=2, $A^3$ = 1,2-Ethylen). Diese Verbindung eignet sich als Komplexbildner zur Komplexierung von Schwermetallen.

Aufgabe der vorliegenden Erfindung war es, Komplexbildner bereitzustellen, welche die Nachteile des Standes der Technik nicht mehr aufweisen.

Demgemäß wurde die eingangs definierte Verwendung der Trisäuren I gefunden.

Die beschriebenen Trisäuren I auf Basis alkoxylierter tertiärer Amine können sowohl in Form der freien Säuren (M = Wasserstoff) als auch in Form ihrer Mono-, Di- oder Trisalze eingesetzt werden.

Als derartige Salze eignen sich vor allem die Natrium-, Kaliumund Ammoniumsalze, insbesondere das Trinatrium-, Trikalium- und Triammoniumsalz sowie organische Triaminsalze mit einem tertiären Stickstoffatom.

Als den organischen Aminsalzen zugrunde liegenden Basen kommen insbesondere tertiäre Amine, wie Trialkylamine mit 1 bis 4 C-Atomen im Alkyl, wie Trimethyl- und Triethylamin, und Trialkanolamine mit 2 oder 3 C-Atomen im Alkanolrest, bevorzugt Triethanolamin, Tri-n-propanolamin oder Triisopropanolamin, in Betracht.

Als 1,2-Alkylengruppen $A^1$ bis $A^3$ kommen vorzugsweise solche mit 2 bis 4 C-Atomen, insbesondere von Ethylenoxid, Propylenoxid oder Butylenoxid abgeleitete Gruppierungen, in Betracht. Vorzugsweise bezeichnen $A^1$ bis $A^3$ dabei 1,2-Ethylen oder 1,2-Propylen. $A^1$ bis $A^3$ können aber auch für größere Gruppierungen stehen, beispielsweise für von Styroloxid oder von längerkettigen Epoxiden wie Decenoxid oder Dodecenoxid abgeleitete Gruppierungen.

Die Variablen x, y und z stehen vorzugsweise unabhängig voneinander für eine Zahl von 1 bis 5, insbesondere 1 bis 3. Besonders bevorzugt werden Trisäuren I, bei denen diese Variablen folgende Bedeutung haben:

$$x = 1, y = 1, z = 1$$

oder

$$x = 2, y = 2, z = 2$$

Bevorzugt werden weiterhin Trisäuren I, bei denen die Variablen $A^1$ bis $A^3$ und/oder $R^1$ bis $R^3$ jeweils gleich sind.

Hinsichtlich der erfindungsgemäßen Verwendung der beschriebenen Trisäuren I sind vor allem die Komplexierung und die Schwermetallkomplexe von Eisen, Kupfer, Zink und Mangan, daneben aber auch von Chrom, Cadmium, Nickel, Silber, Gold und Quecksilber von Interesse. Die Trisäuren I eignen sich darüber hinaus auch als Builder (Gerüststoff) in üblichen Wasch- und Reinigungsmitteln, insbesondere in Textilwaschmittel-Formulierungen.

Die beschriebenen Trisäuren I werden als Schwermetall-Komplexbildner in Bleichbädern in der Papierindustrie, in Wasch- und Reinigungsmitteln, in kosmetischen oder pharmazeutischen Formulierungen, bei der Seifenherstellung, in der Pflanzenernährung sowie zur Herstellung von Metallkomplexen zum Einsatz in galvanischen Bädern oder bei der Rauchgasentschwefelung eingesetzt. In galvanischen Bädern und bei der Rauchgasentschwefelung dienen die auf den Trisäuren I basierenden Metallkomplexe der Veränderung des Redoxpotentials der jeweiligen Metalle.

Eine bevorzugte Verwendung der Trisäuren I ist die in Form der Mangan-Komplexe als Bleichaktivatoren in Wasch- und Reinigungsmitteln, insbesondere in Textilwaschmittel-Formulierungen.

Eine Verwendung für die Trisäuren I und ihre Salze liegt in Bleichbädern in der Papierindustrie. Hier werden Komplexbildner bei der reduktiven Bleiche, z.B. mit Natriumdithionit, oder bei der oxidativen Bleiche, z.B. mit Wasserstoffperoxid, benötigt, um die Effektivität des Bleichprozesses, d.h. den Weißgrad des Holzschliffes zu erhöhen. Die Komplexbildner dienen so zur Eliminierung von Schwermetallkationen, hauptsächlich von Eisen, Kupfer und insbesondere Mangan, die auch bei der Harzleimung mit Alaun und Natriumresinat störend durch die Bildung unlöslicher Salze wirken. Die Ablagerung von Eisen auf Papier führt zu "heißen Flecken", an denen die oxidative katalytische Zerstörung der Zellulose beginnt.

Eine typische Formulierung eines derartigen wäßrigen reduktiven Bleichbades in der Papierindustrie für Holzschliff (beispielsweise 4 % Stoffdichte) enthält 0,05 bis 0,1 Gew.-% Komplexbildner I und ca. 1 Gew.-% Natriumdithionit, jeweils bezogen auf den Holzschliff. Die Badtemperatur beträgt ca. 60°C, die Bleichzeit normalerweise 1 Stunde und der pH-Wert ca. 5,8.

Eine typische Formulierung eines derartigen wäßrigen oxidativen Bleichbades in der Papierindustrie für Holzschliff (beispielsweise 20 % Stoffdichte) enthält 0,05 bis 0,15 Gew.-% Komplexbildner I, ca. 2 Gew.-% Wasserglas, ca. 0,75 Gew.-% NaOH und ca. 1 Gew.-% $H_2O_2$, jeweils bezogen auf den Holzschliff. Die Badtemperatur beträgt ca. 50°C und die Bleichzeit normalerweise 2 Stunden.

In Pharmazeutika und Kosmetika werden die Trisäuren I und ihre Salze hauptsächlich deshalb eingesetzt,

um die schwermetallkatalysierte Oxidation von olefinischen Doppelbindungen und damit das "Ranzigwerden" der Erzeugnisse zu verhindern.

In Seifen verhindern sie schwermetallkatalysierte oxidative Zersetzungen.

Als bevorzugte Verwendung werden in der Pflanzenernährung zur Behebung von Schwermetalldefiziten Kupfer-, Eisen-, Mangan- und Zink-Komplexe der Trisäuren I eingesetzt. Die Schwermetalle werden so als Chelate zugegeben, um die Ausfällung als biologisch inaktive, unlösliche Salze zu verhindern.

Eine Verwendung für die Trisäuren I und ihre Salze liegt in galvanischen Bändern hauptsächlich zur Maskierung von verunreinigenden Schwermetallkationen. Sie dienen hier als Ersatz für die hochtoxischen Cyanide.

Als typische Zusammensetzung eines derartigen wäßrigen galvanischen Bades zur Abscheidung von beispielsweise Kupfer, Nickel, Zink oder Gold sei das folgende Kupferbad angeführt:

| | |
|---|---|
| ca. 10 g/l | Kupfer(II)-sulfat-Pentahydrat |
| 10 bis 12 g/l | Formaldehyd |
| 12 bis 15 g/l | Komplexbildner I |
| 1 bis 2 g/l | eines $C_{13}/C_{15}$-Oxoalkohols, welcher mit 12 mol Ethylenoxid und 6 mol Propylenoxid umgesetzt wurde, als Netzmittel. |

Dieses Bad wird üblicherweise mit Natronlauge auf pH 13 eingestellt; es kann noch übliche Stabilisierungsmittel wie Amine oder Natriumcyanid enthalten.

Eine vorteilhafte Wirkung der Trisäuren I bzw. ihrer Salze liegt in der Bleichmittelstabilisierung, beispielsweise bei der Bleiche von Textilien, Zellstoff oder Papierstoff. Spuren von Schwermetallen wie Eisen, Kupfer und Mangan kommen in den Komponenten des Bleichbades selbst, im Wasser und im zu bleichenden Gut vor und katalysieren die Zersetzung des Bleichmittels. Die Komplexbildner I binden diese Metallionen und verhindern die unerwünschte Zersetzung des Bleichsystems während der Lagerung und bei der Anwendung. Dadurch erhöht sich die Effizienz des Bleichsystems und Schädigungen des zu bleichenden Gutes werden zurückgedrängt.

Die Trisäuren I und ihre Salze eignen sich vor allem deshalb so gut für die beschriebenen Anwendungszwekke, weil sie außerordentlich effektive Komplexbildner für Schwermetallionen, insbesondere für Eisen, Kupfer, Zink und Mangan, darstellen. Ihr Eisen-, Kupfer-, Zink- und Manga.n-Bindevermögen sind außergewöhnlich hoch. Im besonderen kann durch unterschiedliche Einstellungen der Alkoxylierungsgrade x, y und z die Selektivität für einzelne Schwermetallionen gezielt gesteuert werden.

Weitere Vorteile sind ihr sehr geringes Toxizitätspotential und ihre gute biologische Abbaubarkeit. So zeigen die meisten Trisäuren I im Zahn-Wellens-Test unter Standardbedingungen eine biologische Abbaubarkeit > 90 % (28-Tage-Wert), wogegen beispielsweise Ethylendiamintetraessigsäure unter gleichen Bedingungen einen Wert von < 10 % ergibt.

Trisäuren I, bei denen $X^1$ bis $X^3$ Carbonsäuregruppen der Formel COOM bedeuten, die Variablen $R^1$ bis $R^3$ Methylen, 1,1-Ethylen oder 1,1-Propylen bezeichnen und $x \geq 1$ $y \geq 1$ und $z \geq 1$ ist, stellt man vorteilhafterweise dadurch her, daß man Trialkoholamine der allgemeinen Formel IV

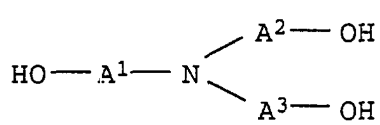

(IV)

mit Alkylenoxid bis zum gewünschten Alkoxylierungsgrad x bzw. y bzw. z umsetzt und die endständigen Hydroxylgruppen katalytisch zu den entsprechenden Carbonsäuren oxidiert. Dieses Verfahren ist deshalb besonders günstig, weil es die Synthese einer Vielzahl von Tricarbonsäuren I ausgehend von kostengünstigen und gut zugänglichen Einsatzstoffen erlaubt. Die Produkte sind zudem chlorfrei und die Ionenselektivität der Komplexbildner läßt sich durch den Alkoxylierungsgrad steuern.

Die Oxidation erfolgt in der Regel in flüssiger Phase mit Luft oder Sauerstoff oder unter dehydrierenden Bedingungen mit Wasserstoff, welcher gegebenenfalls mit Stickstoff verdünnt ist, an einem Kupferkontakt oder an einem Kontakt aus einem Gemisch aus Kupfer und Platinmetallen, d.h. Ruthenium, Rhodium, Osmium, Iridium oder insbesondere Platin oder Palladium, wobei sich der Kontakt vorzugsweise auf Zirkoniumdioxid als Trägermaterial befindet. Man arbeitet hierbei üblicherweise bei Temperaturen von 100 bis 230°C und Drücken von 1 bis 30 bar, vorzugsweise bei 120 bis 200°C und 2 bis 20 bar.

Die Alkoxylierung von IV mit Butylenoxid oder vorzugsweise Ethylenoxid oder Propylenoxid liefert normalerweise statistische Gemische, bei denen die Alkoxylierungsgrade - wie generell in den Verbindungen I - eine statistische Verteilung darstellen. Diese Gemische werden üblicherweise so wie sie anfallen eingesetzt.

Die beschriebene Synthesefolge geht von gut verfügbaren und preiswerten Ausgangsmaterialien aus und liefert die gewünschten Endprodukte in hohen Ausbeuten, Selektivität und Reinheiten. Dabei ist der Anfall an unerwünschten Nebenprodukten, z.B. an anorganischen Salzen, gering.

Synthesebeispiele

Verfahren B: Herstellung der Trisäuren I über die Synthese aus Trialkoholaminen IV

Die Herstellung der benötigten Oxidationskatalysatoren erfolgte nach bekannten Methoden durch Copräzipitation von löslichen Kupferverbindungen wie Kupfernitrat zusammen mit Zirkonylchlorid im alkalischen Medium, Kalzinieren und anschließende Reduktion des Oxids im Wasserstoffstrom.

Beispiel B1: Trisäure-Trinatriumsalz auf Basis von Triethanolamin mit 4 Mol Ethylenoxid

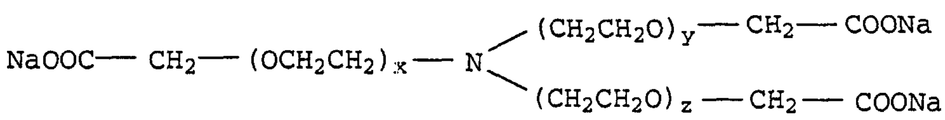

(B1)

x+y+z=4

209 g Triethanolamin und 0,4 g Kalium-tert.-butylat wurden bei 2 mbar Druck für 30 min auf 80°C erwärmt. Danach wurden zu der Mischung unter Druck bei 120°C 246 g Ethylenoxid über 2 h zudosiert. Nach 10 h Reaktionszeit erhielt man 456 g eines Öls mit einer OH-Zahl von 531 (entsprechend 103 % der Theorie).

32,5 g dieses Öls wurden zusammen mit 50 g Wasser, 24,8 g 50 gew.-%iger NaOH und 21,5 g $Cu/ZrO_2$-Katalysator (hergestellt nach obigem Verfahren durch Reduktion von 12 Gew.-% CuO/88 Gew.-% $ZrO_2$ im $H_2$-Strom bei 160°C) in einem Stahlautoklaven vorgelegt. Der Reaktor wurde zweimal mit Stickstoff und einmal mit Wasserstoff gespült, danach wurde für 10 h auf 190°C erhitzt, der entstehende Wasserstoff wurde während der Reaktion abgelassen. Nach dem Abkühlen wurde die Produktlösung vom Katalysator abfiltriert und die Mutterlauge zur Trockne eingeengt. Es verblieben 42,2 g eines farblosen wachsartigen Produktes (entsprechend 97 % der Theorie) mit einem Calciumbindevermögen von 2,17 mmol/g, entsprechend 94 % Wirksubstanz.

Beispiel B2: Trisäure-Trinatriumsalz auf Basis von Triethanolamin mit 8 Mol Ethylenoxid

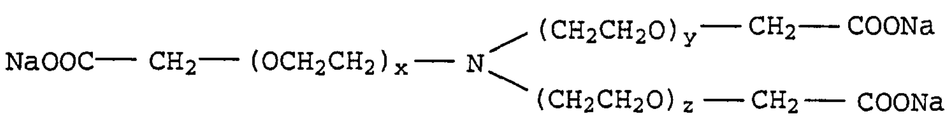

(B2)

x+y+z=8

149 g Triethanolamin und 0,3 g Kalium-tert.-butylat wurden bei 2 mbar Druck für 30 min auf 80°C erwärmt. Danach wurden zu der Mischung unter Druck bei 120°C 352 g Ethylenoxid über 2 h zudosiert. Nach 10 h Reaktionszeit erhielt man 502 g eines Öls mit einer OH-Zahl von 364 (entsprechend 108 % der Theorie).

25,1 g dieses Öls wurden zusammen mit 50 g Wasser, 12,8 g 50 gew.-%iger NaOH und 5,0 g $Cu/ZrO_2$-Katalysator (hergestellt nach obigem Verfahren durch Reduktion von 12 Gew.-% CuO/88 Gew.-% $ZrO_2$ im $H_2$-Strom bei 160°C) in einem Stahlautoklaven vorgelegt. Der Reaktor wurde zweimal mit Stickstoff und einmal mit Wasserstoff gespült, danach wurde für 10 h auf 210°C erhitzt, der entstehende Wasserstoff wurde während der Reaktion abgelassen. Nach dem Abkühlen wurde die Produktlösung vom Katalysator abfiltriert und die Mutterlauge zur Trockne eingeengt. Es verblieben 33,0 g eines farblosen wachsartigen Produktes (entsprechend 108 % der Theorie) mit einem Eisenbindevermögen von 1,36 mmol/g, entsprechend 83 % Wirksubstanz.

Verfahren B1 bzw. B2 lieferten gleiche Ergebnisse, wenn anstelle von Triethanolamin Ammoniak mit 7 bzw. 11 Mol Ethylenoxid umgesetzt und anschließend oxidiert wurde.

## Patentansprüche

**1.** Verwendung von Trisäuren der allgemeinen Formel I

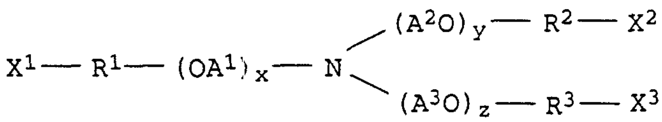

(I)

in der

| | |
|---|---|
| $X^1$ bis $X^3$ | Carbonsäuregruppen der Formel COOM bedeuten, wobei |
| M | für Wasserstoff, Alkalimetall, Ammonium oder substituiertes Ammonium steht, |
| $A^1$ bis $A^3$ | unabhängig voneinander 1,2-Alkylen mit 2 bis 18 C-Atomen bezeichnen, |
| $R^1$ bis $R^3$ | unabhängig voneinander Methylen, 1,1-Ethylen oder 1,1-Propylen bedeuten und |
| x, y und z | unabhängig voneinander für eine Zahl von 1 bis 10 stehen, |

als Schwermetall-Komplexbildner in Bleichbädern in der Papierindustrie, in Wasch- und Reinigungsmitteln, in kosmetischen oder pharmazeutischen Formulierungen, bei der Seifenherstellung, in der Pflanzenernährung sowie zur Herstellung von Schwermetallkomplexen zum Einsatz in galvanischen Bädern oder bei der Rauchgasentschwefelung.

**2.** Verwendung von Trisäuren I nach Anspruch 1 in Form der Mangan-Komplexe als Bleichaktivatoren in Wasch- und Reinigungsmitteln.

**3.** Verwendung von Trisäuren I nach Anspruch 1 oder 2, bei denen $A^1$ bis $A^3$ 1,2-Ethylen oder 1,2-Propylen bezeichnen.

**4.** Verwendung von Trisäuren I nach den Ansprüchen 1 bis 3, bei denen die Variablen x, y und z die Bedeutungen

$$x = 1, y = 1, z = 1$$

oder

$$x = 2, y = 2, z = 2$$

haben.

## Claims

**1.** The use of triacids of the formula I

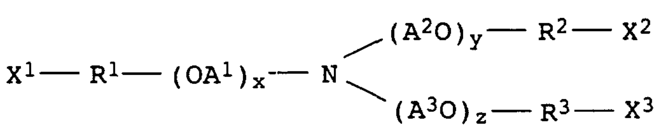

(I)

where

$X^1$ to $X^3$ are carboxylic acid groups of the formula COOM, where

M is hydrogen, alkali metal, ammonium or substituted ammonium,

$A^1$ to $A^3$ are, independently of one another, 1,2-alkylene with 2 to 18 carbon atoms,

$R^1$ to $R^3$ are, independently of one another, methylene, 1,1-ethylene or 1,1-propylene, and

x, y and z are, independently of one another, a number from 1 to 10,

as heavy metal complexing agents in bleaching baths in the paper industry, in detergents and cleaners, in cosmetic or pharmaceutical formulations, in soap manufacture, in crop nutrition and for preparing heavy metal complexes for use in electroplating baths or in the desulfurization of flue gases.

**2.** The use of triacids I as claimed in claim 1 in the form of the manganese complexes as bleach activators in detergents and cleaners.

**3.** The use of triacids I as claimed in claim 1 or 2, where $A^1$ to $A^3$ are 1,2-ethylene or 1,2-propylene.

**4.** The use of triacids I as claimed in any of claims 1 to 3, where the variables x, y and z have the meanings

$$x = 1, y = 1, z = 1$$

or

$$x = 2, y = 2, z = 2.$$

**Revendications**

**1.** Utilisation de triacides répondant à la Formule générale I

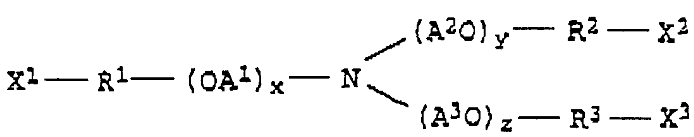

(I)

dans laquelle

$X^1$ à $X^3$ représentent des groupes d'acide carboxylique répondant à la formule COOM, où
M représente un atome d'hydrogène, un métal alcalin, l'ammonium ou l'ammonium substitué,
$A^1$ à $A^3$ désignent, indépendamment les uns des autres, un groupe 1,2-alkylène avec 2 à 18 atomes de carbone,
$R^1$ à $R^3$ signifient, indépendamment les uns des autres, un groupe méthylène, un groupe 1,1-éthylène ou un groupe 1,1-propylène et
x, y et z représentent, indépendamment les uns des autres, un nombre de 1 à 10,

à titre de complexants de métaux lourds dans des bains de blanchiment dans l'industrie du papier, dans des produits de lavage et de nettoyage, dans des formulations cosmétiques ou pharmaceutiques, dans la fabrication de savons, dans la nutrition végétale, de même que pour la préparation de complexes de métaux lourds pour l'utilisation dans des bains de galvanisation, ou dans la désulfuration de gaz de fumée.

**2.** Utilisation de triacides I selon la revendication 1, sous forme des complexes de manganèse à titre d'activateurs de blanchiment dans les produits de lavage et de nettoyage.

**3.** Utilisation de triacides I selon la revendication 1 ou 2, dans lesquels $A^1$ à $A^3$ désignent un groupe 1,2-éthylène ou un groupe 1,2-propylène.

**4.** Utilisation de triacides I selon les revendications 1 à 3, dans lesquels les variables x, y et z présentent les significations

$$x=1, y=1, z=1$$

ou

$$x=2, y=2, z=2.$$